(19) Europäisches Patentamt
European Patent Office
Office européen des brevets

(11) **EP 2 614 368 B1**

(12) **EUROPÄISCHE PATENTSCHRIFT**

(45) Veröffentlichungstag und Bekanntmachung des Hinweises auf die Patenterteilung:
**13.08.2014 Patentblatt 2014/33**

(21) Anmeldenummer: **11726041.4**

(22) Anmeldetag: **14.06.2011**

(51) Int Cl.:
***G01N 33/36*** *(2006.01)*

(86) Internationale Anmeldenummer:
**PCT/CH2011/000146**

(87) Internationale Veröffentlichungsnummer:
**WO 2012/031367 (15.03.2012 Gazette 2012/11)**

(54) **JUSTIERUNG EINER TEXTILEN MESSVORRICHTUNG**

ADJUSTMENT OF A TEXTILE MEASURING DEVICE

RÉGLAGE D'UN DISPOSITIF DE MESURE TEXTILE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.09.2010 CH 144812010**

(43) Veröffentlichungstag der Anmeldung:
**17.07.2013 Patentblatt 2013/29**

(73) Patentinhaber: **Uster Technologies AG**
**8610 Uster (CH)**

(72) Erfinder: **SCHERLER, Philipp**
**CH-8620 Wetzikon (CH)**

(74) Vertreter: **Pliska, Pavel**
**Uster Technologies AG**
**Sonnenbergstrasse 10**
**8610 Uster (CH)**

(56) Entgegenhaltungen:
**WO-A1-2010/043065      DE-A1- 19 535 177**
**US-A- 3 757 211**

## Beschreibung

Die vorliegende Erfindung liegt auf dem Gebiet der textilen Messvorrichtungen. Sie betrifft ein Justierverfahren für eine Vorrichtung zur Untersuchung eines länglichen textilen Prüfgutes gemäss dem Oberbegriff des ersten Patentanspruchs und eine Vorrichtung zur kapazitiven Untersuchung eines länglichen textilen Prüfgutes gemäss dem Oberbegriff eines weiteren unabhängigen Patentanspruchs.

Ein bevorzugtes Einsatzgebiet für die Erfindung ist die kapazitive Untersuchung von länglichen, vorzugsweise textilen Gebilden wie Kardenband, Vorgarn, Garn oder Gewebe. Eine derartige Untersuchung kann bspw. die Detektion von Fremdstoffen, das Erkennen von Änderungen der Masse pro Längeneinheit und/oder die Messung von Feuchtigkeit im Prüfgut zum Ziel haben. Die Erfindung kann bspw. im Produktionsprozess (online) in Gamreinigem auf Spinn- oder Spulmaschinen oder in der Laborprüfung (offline) in Garnprüfgeräten eingesetzt werden.

STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden Sensoren, die auf verschiedenen bekannten Sensorprinzipien basieren. Häufig verwendete Sensorprinzipien sind das kapazitive (siehe z. B. EP-0'924'513 A1) und das optische (siehe z. B. WO-93/13407 A1); bisweilen wird auch das triboelektrische Sensorprinzip eingesetzt (siehe z B. EP-1'037'047 A1). In der vorliegenden Schrift wird hauptsächlich auf das kapazitive Messprinzip eingegangen, weil es sich besonders gut zur Illustration der vorliegenden Erfindung eignet. In kapazitiven Messvorrichtungen wird eine Messschaltung mit einem Messkondensator, der als Plattenkondensator ausgebildet ist, zur Verfügung gestellt. An die Messschaltung wird eine elektrische Wechselspannung angelegt, wodurch im Messkondensator ein elektrisches Wechselfeld erzeugt wird. Das Prüfgut wird durch den Plattenkondensator hindurch bewegt und dem elektrischen Wechselfeld ausgesetzt. Es werden dielektrische Eigenschaften des Prüfgutes ermittelt. Aus den dielektrischen Eigenschaften werden Parameter des Prüfgutes wie Masse pro Längeneinheit und/oder Materialzusammensetzung bestimmt. Beispiele für geeignete Messschaltungen und Auswerteschaltungen für deren Ausgangssignale finden sich in den Schriften EP-0'924'513 A1, WO-2006/105676 A1 und WO-2007/115416 A1.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Messschaltung nebst dem eigentlichen Messkondensator einen Referenzkondensator. Dieser kann z. B. durch Zufügen einer dritten, parallel zu den beiden Messkondensatorplatten angeordneten und elektrisch in Serie zu ihnen geschalteten Kondensatorplatte gebildet werden.

Ohne Prüfgut sollte die Vorrichtung bei angelegter Wechselspannung ein Ausgangssignal null liefern. In der Praxis genügt es jedoch wegen verschiedener Unvollkommenheiten realer elektrischer Komponenten nicht, die Vorrichtung symmetrisch aufzubauen, um ohne Prüfgut ein Nullsignal zu erhalten. Jede einzelne Vorrichtung muss zur Symmetrisierung individuell abgeglichen werden. Die WO-2010/043063 A1 offenbart eine kapazitive Vorrichtung für Garninspektion, die automatisch abgleichbar ist, und ein Verfahren zum Betreiben der Vorrichtung. Die Vorrichtung beinhaltet einen Messkondensator zur Aufnahme des Garns, eine kapazitive Messschaltung und mindestens ein Bauteil mit einer durch ein elektrisches Steuersignal veränderbaren Kapazität. Für den Nullabgleich ist ein geschlossener Regelkreis vorhanden, dessen Regler das Ausgangssignal der kapazitiven Messschaltung auf den Sollwert null regelt. Auch die WO-2010/043065 A1 offenbart eine kapazitive Vorrichtung für Garninspektion mit einem Regelkreis für den Nullabgleich. Der Hauptunterschied zwischen den Lehren der beiden vorgenannten internationalen Veröffentlichungen besteht darin, dass gemäss der ersteren die Abgleichmittel in der Messschaltung selbst eingebaut sind, während gemäss der letzteren die Abgleichmittel ausserhalb der Messschaltung liegen.

Garnreiniger auf Spinn- oder Spulmaschinen werden virtuell zu Gruppen zusammengefasst, von denen jede bis zu einigen Dutzend Garnreinigermessköpfe beinhalten kann. Alle Garnreinigermessköpfe innerhalb derselben Gruppe haben dieselben Einstellungen. Einander entsprechende Messungen der Garnreinigermessköpfe werden teilweise miteinander verglichen, um Ausreisser zu finden. So misst z. B. jeder Garnreinigermesskopf die Garnnummer, d. h. die Länge pro Masse des Garns, und wenn die Garnnummemmessung eines Garnreinigermesskopfes signifikant von den Messungen der übrigen Garnreinigermessköpfe derselben Gruppe abweicht, so wird ein Garnnummernfehler registriert und ein Warnsignal ausgegeben. Um solche vergleichenden Messungen durchführen zu können, müssen die Granreinigermessköpfe absolut messen oder zumindest gleich justiert sein. Die Justierung berücksichtigt geringfügige Unterschiede in den eingesetzten elektronischen Bauteilen und wird vor der Auslieferung des Garnreinigermesskopfes einmal werkseitig vorgenommen. Sie resultiert in einem Justierwert, der in im Garnreinigermesskopf gespeichert und während seiner ganzen Lebensdauer nicht mehr verändert wird. Die Erfahrung zeigt aber, dass sich Eigenschaften der elektronischen Bauteile langfristig verändern können, etwa durch Alterungs- oder Abnützungsprozesse. Dadurch verändert sich auch die Empfindlichkeit des Garnreinigermesskopfes. Dies führt zu Messungenauigkeiten und zu falschen Fehlermeldungen.

Die oben beschriebenen Vorgänge des Nullabgleichs und der Justierung müssen gut voneinander unterschieden werden. Der Nullabgleich dient, vereinfacht gesagt, der Festlegung des Nullpunkts für eine Messung. Der Nullabgleich ist von grundlegender Bedeutung für jede Messung, denn ohne vorherige Festlegung des Nullpunkts sind die Messresultate ohne Aussagekraft. Im Unterschied dazu dient die Justierung, so wie der Begriff in der vorliegenden Schrift verwendet wird, der Streckung oder Stauchung einer Messskala, wobei der vorher festgelegte Nullpunkt als Fixpunkt dient. Auf die Justierung könnte verzichtet werden, wenn nur Messresultate von ein und derselben Messvorrichtung, die alle innerhalb einer kurzen bis mittleren Zeitspanne erhalten wurden, miteinander verglichen würden. Die Justierung wird erst dann wichtig, wenn Messresultate verschiedener Messvorrichtungen oder zeitlich weit auseinander liegende Messungen miteinander verglichen werden sollen, oder wenn Absolutmessungen vorgenommen werden sollen. Die vorliegende Erfindung befasst sich mit ebendieser Justierung.

DARSTELLUNG DER ERFINDUNG

Es ist deshalb eine Aufgabe der vorliegenden Erfindung, ein Verfahren zur automatischen Justierung einer Vorrichtung zur Untersuchung eines länglichen textilen Prüfgutes anzugeben. Eine weitere Aufgabe ist es, eine Vorrichtung zur Untersuchung eines länglichen textilen Prüfgutes zu schaffen, die automatisch justiert werden kann.

Diese und andere Aufgaben werden durch das erfindungsgemässe Justierverfahren und die erfindungsgemässe Vorrichtung, wie sie in den unabhängigen Patentansprüchen definiert sind, gelöst. Vorteilhafte Ausführungsformen sind in den abhängigen Patentansprüchen angegeben.

Ausgangspunkt für die kapazitive Ausführungsform der vorliegenden Erfindung war die WO-2010/043063 A1. Die dort beschriebene kapazitive Messschaltung beinhaltet mindestens ein Bauteil mit einer durch ein elektrisches Steuersignal veränderbaren Kapazität. Im Unterschied zur WO-2010/043063 A1 wird aber gemäss der vorliegenden Erfindung das Bauteil nicht zum Nullabgleich, sondern zur Justierung genutzt. Beim Justieren wird die Messschaltung durch Anlegen eines elektrischen Steuersignals gezielt verstimmt, und ein elektrisches Ausgangssignal wird gemessen. Bei einem gegebenen Steuersignal ist das Ausgangssignal ein Mass für die Empfindlichkeit oder Verstärkung der Messschaltung und kann daher zur Justierung verwendet werden. Der ganze Justiervorgang läuft vorzugsweise automatisch ab. Obwohl die kapazitive Ausführungsform bevorzugt wird, gibt es auch andere Sensorprinzipien, mit denen die Erfindung ausführbar ist, so z. B. das optische oder das triboelektrische.

Das erfindungsgemässe Justierverfahren dient der Justierung einer Vorrichtung zur Untersuchung eines länglichen textilen Prüfgutes. Die Vorrichtung beinhaltet eine durch mindestens ein elektrisches Steuersignal verstimmbare Messschaltung mit einem Sensor für mindestens eine Eigenschaft des Prüfgutes. Die Messschaltung wird durch das mindestens eine elektrische Steuersignal derart verstimmt, dass sich eine durch die Verstimmung verursachte, von null verschiedene Änderung eines Ausgangssignals der Messschaltung bezüglich eines Abgleichwertes ergibt. Die durch die Verstimmung verursachte Änderung des Ausgangssignals wird ermittelt. Das verstimmende Steuersignal sowie die ermittelte Änderung des Ausgangssignals werden rechnerisch miteinander verknüpft. Ein Justierwert wird aus einem Resultat der Verknüpfung berechnet und in einer Speichereinheit der Vorrichtung gespeichert.

Die rechnerische Verknüpfung beinhaltet vorzugsweise eine Quotientenbildung. Dies ist deshalb sinnvoll, weil die beiden miteinander verknüpften Grössen innerhalb des Arbeitsbereichs annähernd proportional zueinander sind, ihr Quotient also konstant ist. Der Quotient aus der Änderung des Ausgangssignals und dem verstimmenden Steuersignal entspricht einer Verstärkung, welche in den Justierwert eingeht.

Der Justierwert ist bspw. proportional oder umgekehrt proportional zu einem Verhältnis aus der ermittelten Änderung des Ausgangssignals und der erzeugten Änderung des Steuersignals. Anlässlich einer ersten Justierung, die mit einer Massverkörperung erfolgt, werden ein erster Justierwert sowie das besagte Verhältnis ermittelt, und anlässlich aller nachfolgenden Justierungen wird das besagte Verhältnis neu ermittelt und daraus ein neuer Justierwert berechnet.

Der genannte Abgleichwert ist derjenige Wert, den das Ausgangssignal der Messschaltung, annimmt, wenn die Messschaltung abgeglichen und kein Prüfgut eingelegt ist. Ein automatisches Abgleichverfahren für eine kapazitive Messschaltung ist in der WO-2010/043063 A1 beschrieben. Der Abgleichwert ist vorzugsweise gleich null.

Das Prüfgut ist z. B. ein Garn. Die Justierung erfolgt erfindungsgemäß automatisch, und zwar dann, wenn sich kein Prüfgut im Messbereich des Sensors befindet.

Ebenso sollte darauf geachtet werden, dass während der Justierung die Umgebungsbedingungen wie Temperatur und Luftfeuchtigkeit stabil bleiben und denjenigen entsprechen, die bei der Untersuchung vorherrschen.

Der Sensor kann ein kapazitiver, ein optischer und/oder ein triboelektrischer Sensor sein. In einer besonders bevorzugten Ausführungsform mit einem kapazitiven Sensor ist der Sensor ein Messkondensator zur Aufnahme des Prüfgutes, die Messschaltung beinhaltet mindestens ein Bauteil mit einer durch mindestens ein elektrisches Steuersignal veränderbaren Kapazität, die Kapazität wird durch das mindestens eine elektrische Steuersignal verändert und eine durch diese Veränderung verursachte Änderung eines Ausgangssignals der Messschaltung wird ermittelt. Die Vorrichtung beinhaltet vorzugsweise zwei Bauteile mit jeweils einer durch mindestens ein elektrisches Steuersignal veränderbaren Kapazität, und die Kapazitäten werden im Justierverfahren im Wesentlichen antisymmetrisch zueinander verändert.

Eine bevorzugte Anwendung des erfindungsgemässen Justierverfahrens ist das Justieren eines Garnreinigers.

Die erfindungsgemässe Vorrichtung zur Untersuchung eines länglichen textilen Prüfgutes beinhaltet eine durch mindestens ein elektrisches Steuersignal verstimmbare Messschaltung mit einem Sensor für mindestens eine Eigenschaft des Prüfgutes. Ferner beinhaltet die Vorrichtung eine Auswerteeinheit, die zur Ausführung des oben beschriebenen erfindungsgemässen Justierverfahrens eingerichtet ist.

Der Sensor kann ein kapazitiver, ein optischer und/oder ein triboelektrischer Sensor sein. In einer besonders bevorzugten Ausführungsform mit einem kapazitiven Sensor ist der Sensor ein Messkondensator zur Aufnahme des Prüfgutes, und die Messschaltung beinhaltet mindestens ein Bauteil mit einer durch das mindestens eine elektrische Steuersignal veränderbaren Kapazität.

Die Messschaltung kann als Kapazitätsmessbrücke mit mindestens zwei Brückenzweigen ausgebildet sein. Ein erster Brückenzweig ist ein Messzweig, der den Messkondensator beinhaltet. Ein zweiter Brückenzweig beinhaltet ein Bauteil mit einer durch mindestens ein elektrisches Steuersignal veränderbaren Kapazität.

Das mindestens eine Bauteil ist ein Bauteil aus der folgenden Menge: Kapazitätsdiode, andere Diode, eine Mehrzahl von zueinander parallel geschalteten Abgleichkondensatoren, die mit entsprechenden elektrischen Steuersignalen zu- und wegschaltbar sind, insbesondere MEMS-Kondensatoren, in Sperrrichtung betriebener Transistor, MetallOxid-Halbleiter-Feldeffekttransistor, mikro-elektromechanischer Kondensator, Kondensator, dessen Elektrodenabstand mittels eines Piezoelementes veränderbar ist.

Die Messschaltung ist vorzugsweise als Kapazitätsvollmessbrücke mit zwei Brückenzweigen, die nicht durch einen Brückenstamm miteinander verbunden sind, ausgebildet. Der erste Brückenzweig beinhaltet den Messkondensator und einen Referenzkondensator, und der zweite Brückenzweig beinhaltet zwei Bauteile mit jeweils einer durch mindestens ein elektrisches Steuersignal veränderbaren Kapazität. Die Auswerteeinheit kann zur Ermittlung einer Differenz zwischen einem ersten analogen Ausgangssignal, das am ersten Brückenzweig an einer Verbindungsstelle zwischen dem Messkondensator und dem Referenzkondensator abgegriffen wird, und einem zweiten analogen Ausgangssignal, das am zweiten Brückenzweig an einer Verbindungsstelle zwischen den beiden Bauteilen abgegriffen wird, eingerichtet sein.

Die Vorrichtung kann ferner Mittel zum Anlegen eines elektrischen Wechselsignals an die Messschaltung zwecks Erzeugung eines elektrischen Wechselfeldes im Messkondensator und eine Durchgangsöffnung für das Prüfgut im Messkondensator, welche Durchgangsöffnung vom elektrischen Wechselfeld beaufschlagbar ist, beinhalten.

Die Vorrichtung wird vorzugsweise zur Untersuchung eines länglichen textilen Prüfgutes wie Kardenband, Vorgarn, Garn oder Gewebe verwendet, wobei das durch die Vorrichtung bewegte Prüfgut vom Sensor abgetastet wird. Eine besonders bevorzugte Anwendung ist die Garnreinigung. Diesfalls ist das Prüfgut ein Garn und die Vorrichtung ein Garnreiniger, der z. B. auf einer Spinn- oder Spulmaschine einsetzbar ist.

Die erfindungsgemässe Justierung kompensiert individuelle Unterschiede von Vorrichtungen zur Untersuchung eines länglichen textilen Prüfgutes. Sie macht Messungen mehrerer Vorrichtungen, etwa mehrerer zu einer Gruppe zusammengefasster Garnreiniger auf einer Spulmaschine, miteinander vergleichbar. Dadurch steigert sie die Zuverlässigkeit beim Identifizieren von Ausreissern, etwa Garnnummernfehlern, und erhöht die Produktivität. Sie ermöglicht auch die Durchführung von Absolutmessungen.

AUFZÄHLUNG DER ZEICHNUNGEN

Nachfolgend wird eine bevorzugte Ausführungsform der Erfindung anhand der Zeichnungen detailliert erläutert.

Figur 1   zeigt ein Blockschema einer kapazitiven Ausführungsform der erfindungsgemässen Vorrichtung.

Figur 2   zeigt ein Flussdiagramm einer Ausführungsform des erfindungsgemässen Verfahrens.

AUSFÜHRUNG DER ERFINDUNG

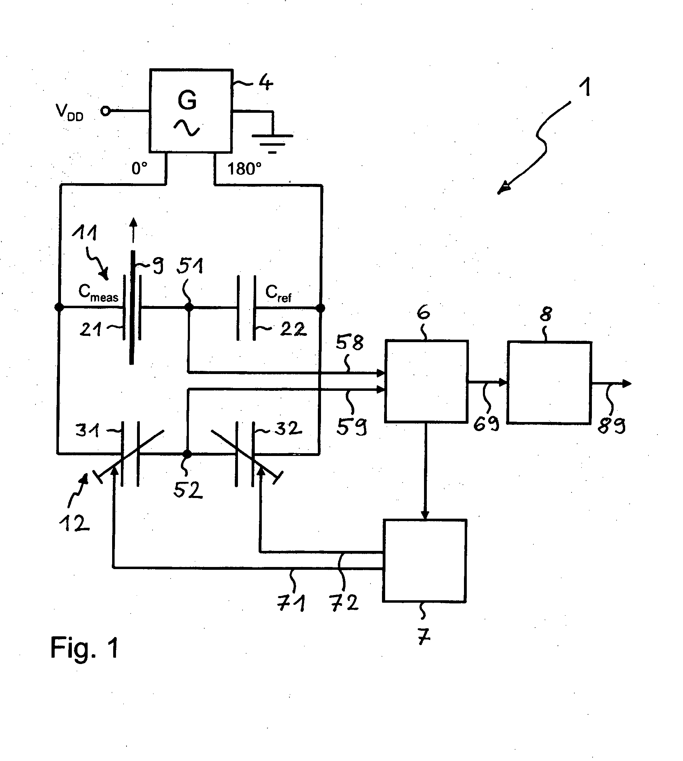

Figur 1 zeigt eine Ausführungsform einer erfindungsgemässen kapazitiven Messvorrichtung 1. Diese beinhaltet eine Messschaltung, die als Kapazitätsmessbrücke ausgebildet ist. Sie umfasst zwei parallel zueinander geschaltete Brückenzweige 11, 12, die jeweils mindestens zwei in Serie zueinander geschaltete Kapazitäten aufweisen. Ein Wechselsignalgenerator 4 erhält eine Speisespannung $V_{DD}$ und legt an der Kapazitätsmessbrücke zwei elektrische Wechselsignale an, die gegeneinander um 180° phasenverschoben sind. Zwei Ausgangsanschlüsse 51, 52 greifen analoge Ausgangssignale der Kapazitätsmessbrücke jeweils zwischen den beiden in Serie zueinander geschalteten Kapazitäten ab. Die Ausgangssignale werden auf analogen Ausgangsleitungen 58, 59 in eine Auswerteeinheit 6 mit differenziellem Eingang geleitet, wo sie ausgewertet werden. Die Auswerteeinheit 6 gibt auf einer Zwischenleitung 69 ein digitales Zwischensignal aus, das in einer Justiereinheit 8 in ein justiertes digitales Ausgangssignal umgerechnet und auf einer digitalen Ausgangsleitung 89 ausgegeben wird.

In einem ersten Brückenzweig 11 befindet sich ein Messkondensator 21, der zur Aufnahme eines in seiner Längsrichtung bewegten länglichen textilen Prüfgutes 9 wie Kardenband, Vorgarn, Garn oder Gewebe geeignet ist. Ein mit dem Messkondensator 21 in Serie geschalteter Kondensator 22 kann als Referenzkondensator vorgesehen sein. Der Referenzkondensator 22 ermöglicht genaue, von äusseren Einflüssen wie Lufttemperatur oder Luftfeuchtigkeit nicht beeinflusste Kompensationsmessungen am Prüfgut 9. Der Messkondensator 21 und der Referenzkondensator 22 sollten möglichst identisch sein, um auch möglichst gleiche Kapazitäten aufzuweisen. Die beiden einander zugewandten Kondensatorplatten des Messkondensators 21 bzw. des Referenzkondensators 22, die auf demselben elektrischen Potenzial liegen, können als eine einzige Kondensatorplatte realisiert sein, wodurch eine Quelle von möglichen Asymmetrien beseitigt wird.

Ein zweiter Brückenzweig 12 weist Bauteile 31, 32 mit veränderbaren Kapazitäten auf. Die Bauteile 31, 32 können z. B. als Kapazitätsdioden (auch Kapazitäts-Variationsdiode oder Varicap genannt) realisiert sein. Die Bauteile 31, 32 werden durch je eine analoge elektrische Steuerspannung auf Steuerleitungen 71, 72 gesteuert. In einer bevorzugten Ausführungsform sind die Steuerspannungen der beiden Bauteile 31, 32 bezüglich einer Referenzspannung zueinander antisymmetrische Gleichspannungen, d. h. ihre Differenzen zur Referenzspannung sind betragsmässig gleich und haben entgegengesetzte Vorzeichen. Die Steuerspannungen werden von einer Steuereinheit 7 geliefert, die ihrerseits von der Auswerteeinheit 6 gesteuert wird.

In einer Weiterbildung der Ausführungsform von Figur 1 können der Messkondensator 21 und der Referenzkondensator 22, die zusammen als eine Messeinheit angesehen werden können, mehrfach vorhanden sein. Die weiteren derartigen Messeinheiten sind dann parallel zur in Figur 1 eingezeichneten ersten Messeinheit 21, 22 geschaltet. Sie beinhalten vorzugsweise Kondensatoren mit unterschiedlichen Kapazitäten. Diese können zur Ausmessung von unterschiedlichem Prüfgut 9, z. B. von verschieden dicken Garnen, verwendet werden. Die Unterscheidung von Mess- und Referenzkondensator dient in dieser Schrift eher einer eindeutigen Namensgebung als einer starren Zuweisung von Funktionen, denn in vielen Fällen kann ein Messkondensator auch als Referenzkondensator dienen und umgekehrt. Sind in einer Messschaltung mehrere Mess- und Referenzkondensatoren vorhanden, so dient in der Regel bei einer Messung einer der Kondensatoren als Messkondensator und die übrigen Kondensatoren als Referenzkondensatoren.

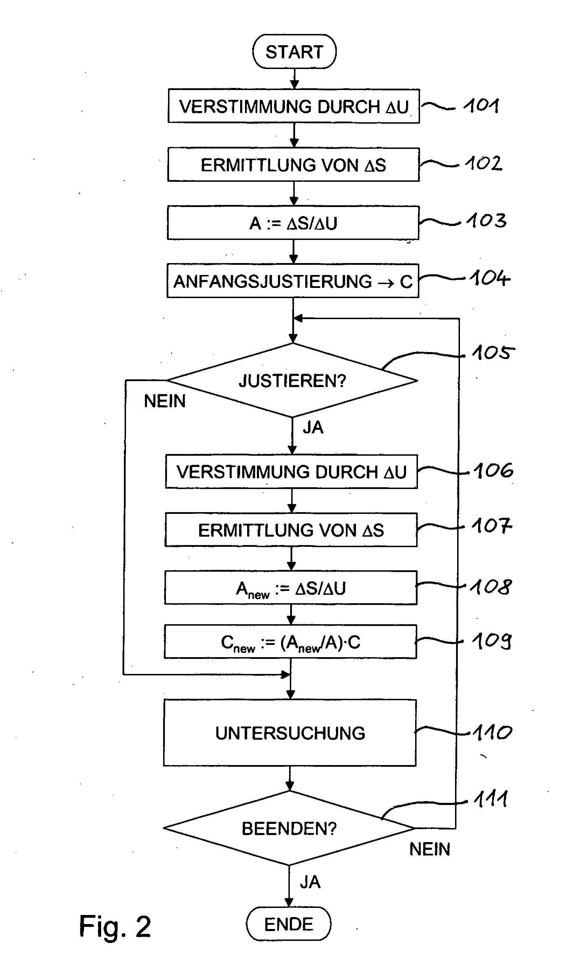

Das erfindungsgemässe Justierverfahren wird anhand des Flussdiagramms von Figur 2 erläutert, wobei die Strukturen, auf die Bezug genommen wird, in Figur 1 dargestellt sind. Vor der Auslieferung an einen Kunden wird die erfindungsgemässe Vorrichtung 1 vom Hersteller ein erstes Mal justiert. Das erfindungsgemässe Justierverfahren wird vorzugsweise dann durchgeführt, wenn sich kein Prüfgut 9 im Messkondensator 21 befindet. Während jeder Justierung sollte auch auf stabile Umgebungsbedingungen wie Temperatur und Luftfeuchtigkeit geachtet werden.

Jeder Justierung sollte vorzugsweise ein Nullabgleich der Messvorrichtung 1 vorangehen, wie er z. B. in der WO-2010/043063 A1 beschrieben ist. Aus dem Nullabgleich ergibt sich ein Abgleichwert, der als Basis für das erfindungsgemässe Verfahren benötigt wird. Ebenso sollte ein Nullabgleich vor einer neuen Untersuchung vorgenommen werden, wenn unmittelbar zuvor kein Justiervorgang stattfand. Wie einleitend beschrieben, ist der Nullabgleich von grundlegender Bedeutung für jede Verwendung der Messvorrichtung 1.

Eine Anfangsjustierung 101-104 beinhaltet eine Ermittlung einer Verstärkung A (Schritte 101-103) und eine Bestimmung eines Justierwertes C (Schritt 104).

Im abgeglichenen Zustand gibt die Auswerteeinheit 6 (siehe Figur 1) auf der Zwischenleitung 69 ein konstantes Abgleichsignal, vorzugsweise ein Nullsignal, aus. Zur Ermittlung der Verstärkung A wird die Kapazitätsmessbrücke gezielt verstimmt 101, indem die Kapazitäten der veränderbaren Bauteile 31, 32 durch ein elektrisches Steuersignal $\pm\Delta U$ von der Steuereinheit 7 verändert werden. Durch diese Verstimmung 101 ändert sich das Zwischensignal auf der Zwischenleitung 69 vom ursprünglichen Abgleichwert, z. B. null, auf einen anderen Wert. Die durch die Verstimmung 101 verursachte Änderung $\Delta S$ des Zwischensignals ist von null verschieden und wird ermittelt 102. Aus den beiden Signalen $\Delta U$, $\Delta S$ kann eine Verstärkung A durch Quotientenbildung berechnet 103 und in der Justiereinheit 8 gespeichert werden:

$$A := \Delta S / \Delta U \ .$$

Bei der werkseitigen Anfangsjustierung wird eine (nicht eingezeichnete) Massverkörperung in den Messkondensator 21 eingelegt und ein Anfangsjustierwert C bestimmt 104. Der Justierwert C wird bei der späteren Verwendung 110 der Vorrichtung 1 dazu gebraucht, um aus dem Zwischensignal auf der Zwischenleitung 69 ein Ausgangssignal der gewünschten Dimension auf der Ausgangsleitung 89 zu erhalten, bspw. die Garnnummer in tex. Typischerweise wird dabei das gemessene Zwischensignal durch den Justierwert C dividiert oder mit diesem multipliziert. Die Verstärkung A und der Anfangsjustierwert C werden in einem Speicher der Justiereinheit 8 abgelegt.

Mit der Bestimmung 104 und Speicherung des Justierwertes C ist die Anfangsjustierung 101-104 abgeschlossen, und die Vorrichtung 1 kann an einen Kunden ausgeliefert werden. Dieser wird mit der Vorrichtung 1 bestimmungsgemäss Untersuchungen 110 an länglichem textilem Prüfgut 9 wie Garn vornehmen. Der Justierwert C hängt von verschiedenen Einflüssen ab, z. B. vom Typ der in der Messschaltung verwendeten elektrischen Komponenten, ihrem Alter und ihrer Abnützung, aber auch von äusseren Einflüssen wie der Temperatur und der Luftfeuchtigkeit. Deshalb ist eine bloss einmalige, werkseitige Anfangsjustierung für zuverlässige, wiederholbare und vergleichbare Absolutmessungen oft ungenügend.

Ein neuer Justiervorgang 106-109 kann automatisch dann ausgelöst werden 105, wenn sich kein Prüfgut 9 im Messkondensator 21 befindet. Bei einem Garnreiniger ist dies z. B. während eines Kopswechsels oder nach einem Reinigerschnitt der Fall. Analog zur Anfangsjustierung 101-104 wird zunächst eine neue Verstärkung $A_{new}$ ermittelt. Zu diesem Zweck wird die zuvor auf einen Abgleichwert abgeglichene Kapazitätsmessbrücke durch das Steuersignal $\Delta U$ verstimmt 106. Die Verstimmung 106 verursacht eine von null verschiedene Änderung $\Delta S$ des Zwischensignals auf der Zwischenleitung 69 bezüglich des Abgleichwertes. Es wird die Änderung $\Delta S$ des Zwischensignals ermittelt 107 und die neue Verstärkung $A_{new}$ als Verhältnis des Zwischensignals $\Delta S$ und des Steuersignals $\Delta U$ berechnet 108. Da der Justierwert C proportional ist zur Verstärkung A, kann aus den alten, in der Justiereinheit 8 gespeicherten Werten C sowie A und aus der neu berechneten Verstärkung $A_{new}$ ein neuer Justierwert $C_{new}$ gemäss der folgenden Formel berechnet werden 109:

$$C_{new} := (A_{new}/A) \cdot C \ .$$

Die so neu bestimmten Werte $A_{new}$ und $C_{new}$ ersetzen die alten Werte A bzw. C im Speicher der Justiereinheit 8. Die neue Justierung 106-109 ist damit abgeschlossen. Nach der Justierung 106-109 kann die Untersuchung 110 des länglichen textilen Prüfgutes 9 aufgenommen bzw. fortgesetzt werden. Dabei wird der aktuelle, in der Justiereinheit 8 gespeicherte Justierwert $C_{new}$ verwendet. Wenn die Untersuchung 110 abgeschlossen ist, kann die Vorrichtung 1 angehalten werden 111, oder es kann zu einem geeigneten Zeitpunkt ein neuer Justiervorgang 106-109 ausgelöst werden 105.

Selbstverständlich ist die vorliegende Erfindung nicht auf die oben diskutierten Ausführungsformen beschränkt. Insbesondere sei betont, dass die oben beispielhaft diskutierte kapazitive Ausführungsform die Allgemeinheit der Erfindung nicht einschränken soll. Die Erfindung ist ebenso mit anderen Sensoren ausführbar, z. B. mit einem optischen Sensor. Im letzteren Fall kann die Verstimmung 101, 106 bspw. durch eine Änderung eines an einer Lichtquelle angelegten elektrischen Signals erfolgen. Die anlässlich der bevorzugten kapazitiven Ausführungsform beschriebene Verstimmung kann anders als mittels veränderbarer Kapazitäten erfolgen, z. B. mittels veränderbarer Widerstände. Die in Figur 1 vorgenommene Aufteilung in Auswerteinheit 6, Steuereinheit 7 und Justiereinheit 8 entspricht den Funktionen, aber nicht notwendigerweise den physischen Strukturen. Dieselben Funktionen können in anderer Weise auf elektronische Bauteile aufgeteilt oder von einem einzigen Bauteil wahrgenommen werden. Bei Kenntnis der Erfindung wird der Fachmann weitere Varianten herleiten können, die auch zum Gegenstand der vorliegenden Erfindung gehören.

BEZUGSZEICHENLISTE

| 1 | Vorrichtung |
|---|---|
| 11, 12 | Brückenzweige |
| 21 | Messkondensator |
| 22 | Referenzkondensator |
| 31, 32 | Bauteile mit veränderbarer Kapazität |
| 4 | Wechselsignalgenerator |
| 51, 52 | Ausgangsanschlüsse |
| 58, 59 | analoge Ausgangsleitungen |
| 6 | Auswerteeinheit |
| 69 | Zwischenleitung |
| 7 | Steuereinheit |
| 71, 72 | Steuerleitungen |
| 8 | Justiereinheit |
| 89 | digitale Ausgangsleitung |
| 9 | Prüfgut |
| 101, 106 | Verstimmung der Messschaltung |
| 102, 107 | Ermittlung einer Änderung des Ausgangssignals |
| 103, 108 | Berechnung einer Verstärkung |
| 104 | Anfangsjustierung |
| 105 | Entscheidung über Justierung |
| 109 | Berechnung eines neuen Justierwertes |
| 110 | Untersuchung des Prüfgutes |
| 111 | Entscheidung über Ende der Untersuchung |

**Patentansprüche**

1. Justierverfahren für eine Vorrichtung (1) zur Untersuchung eines länglichen textilen Prüfgutes (9), welche Vorrichtung (1) eine durch mindestens ein

elektrisches Steuersignal (∆U) verstimmbare Messschaltung mit einem Sensor (21) für mindestens eine Eigenschaft des Prüfgutes (9) beinhaltet,
wobei
die Messschaltung durch das mindestens eine elektrische Steuersignal (∆U) derart verstimmt (101, 106) wird, dass sich eine durch die Verstimmung (101, 106) verursachte, von null verschiedene Änderung (∆S) eines Ausgangssignals der Messschaltung bezüglich eines Abgleichwertes ergibt,
**dadurch gekennzeichnet, dass**
die durch die Verstimmung (101, 106) verursachte Änderung (∆S) des Ausgangssignals ermittelt wird (102, 107),
das verstimmende Steuersignal (∆U) sowie die ermittelte Änderung (∆S) des Ausgangssignals rechnerisch miteinander verknüpft werden (103, 108),
ein Justierwert (C) aus einem Resultat der Verknüpfung (103, 108) berechnet (104, 109) sowie in einer Speichereinheit der Vorrichtung (1) gespeichert wird und
die Justierung dann automatisch erfolgt, wenn sich kein Prüfgut (9) im Messbereich des Sensors (21) befindet.

2. Justierverfahren nach Anspruch 1, wobei die rechnerische Verknüpfung (103, 108) eine Quotientenbildung beinhaltet.

3. Justierverfahren nach einem der vorangehenden Ansprüche, wobei der Justierwert (C) proportional oder umgekehrt proportional ist zu einem Verhältnis aus der ermittelten Änderung des Ausgangssignals (∆S) und der erzeugten Änderung des Steuersignals (∆U).

4. Justierverfahren nach Anspruch 3, wobei anlässlich einer ersten Justierung (101-104), die mit einer Massverkörperung erfolgt, ein erster Justierwert (C) sowie das besagte Verhältnis (∆S/∆U) ermittelt werden und anlässlich aller nachfolgenden Justierungen (106-109) das besagte Verhältnis (∆S/∆U) neu ermittelt und daraus ein neuer Justierwert ($C_{new}$) berechnet wird.

5. Justierverfahren nach einem der vorangehenden Ansprüche, wobei der Abgleichwert gleich null ist.

6. Justierverfahren nach einem der vorangehenden Ansprüche, wobei das Prüfgut (9) ein Garn ist.

7. Justierverfahren nach Anspruch 6, wobei
der Sensor ein Messkondensator (21) zur Aufnahme des Prüfgutes (9) ist,
die Messschaltung mindestens ein Bauteil (31, 32) mit einer durch mindestens ein elektrisches Steuersignal (∆U) veränderbaren Kapazität beinhaltet,
die Kapazität durch das mindestens eine elektrische

Steuersignal (∆U) verändert wird (106) und eine durch diese Veränderung (106) verursachte Änderung (∆S) eines Ausgangssignals der Messschaltung ermittelt wird (107).

8. Justierverfahren nach Anspruch 7, wobei die Vorrichtung (1) zwei Bauteile (31, 32) mit jeweils einer durch mindestens ein elektrisches Steuersignal (±∆U) veränderbaren Kapazität beinhaltet und die Kapazitäten im Wesentlichen antisymmetrisch zueinander verändert werden (106).

9. Anwendung des Justierverfahrens nach einem der vorangehenden Ansprüche zum Justieren eines Garnreinigers.

10. Vorrichtung (1) zur Untersuchung eines länglichen textilen Prüfgutes (9), beinhaltend
eine durch mindestens ein elektrisches Steuersignal (∆U) verstimmbare Messschaltung mit einem Sensor (21) für mindestens eine Eigenschaft des Prüfgutes (9),
**gekennzeichnet durch**
eine Auswerteeinheit (6), die zur Ausführung des Justierverfahrens nach einem der vorangehenden Ansprüche eingerichtet ist.

11. Vorrichtung (1) nach Anspruch 10, wobei der Sensor (21) ein kapazitiver, ein optischer und/oder ein triboelektrischer Sensor ist.

12. Vorrichtung (1) nach Anspruch 11, wobei
der Sensor ein Messkondensator (21) zur Aufnahme des Prüfgutes (9) ist und die Messschaltung mindestens ein Bauteil (31, 32) mit einer durch das mindestens eine elektrische Steuersignal (∆U) veränderbaren Kapazität beinhaltet.

13. Vorrichtung (1) nach Anspruch 12, wobei
die Messschaltung als Kapazitätsmessbrücke mit mindestens zwei Brückenzweigen (11, 12) ausgebildet ist, von denen
ein erster Brückenzweig (11) ein Messzweig ist, der den Messkondensator (21) beinhaltet und
ein zweiter Brückenzweig (12) ein Bauteil (31, 32) mit einer durch mindestens ein elektrisches Steuersignal (∆U) veränderbaren Kapazität beinhaltet.

14. Vorrichtung (1) nach Anspruch 12 oder 13, ferner beinhaltend
Mittel (4) zum Anlegen eines elektrischen Wechselsignals an die Messschaltung zwecks Erzeugung eines elektrischen Wechselfeldes im Messkondensator (21), und
eine Durchgangsöffnung für das Prüfgut (9) im Messkondensator (21), welche Durchgangsöffnung vom elektrischen Wechselfeld beaufschlagbar ist.

**15.** Vorrichtung (1) nach einem der Ansprüche 10-14, wobei die Vorrichtung (1) ein Garnreiniger ist.

**Claims**

**1.** An adjustment method for a device (1) for examining an elongate textile test material (9), which device (1) contains a measuring circuit which is detunable by at least one electrical control signal ($\Delta$U), having a sensor (21) for at least one property of the test material (9), wherein the measuring circuit is detuned (101, 106) by the at least one electrical control signal ($\Delta$U) such that a change ($\Delta$S), which is caused by the detuning (101, 106) and is not equal to zero, of an output signal of the measuring circuit in relation to a balancing value results,
**characterized in that**
the change ($\Delta$S) of the output signal caused by the detuning (101, 106) is determined (102, 107), the detuned control signal ($\Delta$U) and the determined change ($\Delta$S) of the output signal are linked to one another (103, 108) arithmetically, an adjustment value (C) is calculated (104, 109) from a result of the linkage (103, 108) and stored in a storage unit of the device (1) and the adjustment is performed automatically when no test material (9) is located in the measuring region of the sensor (21).

**2.** The adjustment method according to claim 1, wherein the arithmetical linkage (103, 108) includes a quotient calculation.

**3.** The adjustment method according to one of the preceding claims, wherein the adjustment value (C) is proportional or inversely proportional to a ratio of the determined change of the output signal ($\Delta$S) and the generated change of the control signal ($\Delta$U).

**4.** The adjustment method according to claim 3, wherein in the case of a first adjustment (101-104), which is performed with a mass embodiment, a first adjustment value (C) and the mentioned ratio ($\Delta$S/$\Delta$U) are determined and in the case of all subsequent adjustments (106-109), the mentioned ratio ($\Delta$S/$\Delta$U) is determined again and a new adjustment value ($C_{new}$) is calculated therefrom.

**5.** The adjustment method according to one of the preceding claims, wherein the balancing value is equal to zero.

**6.** The adjustment method according to one of the preceding claims, wherein the test material (9) is a yarn.

**7.** The adjustment method according to claim 6, wherein
the sensor is a measuring capacitor (21) for accommodating the test material (9), the measuring circuit contains at least component (31, 32) having a capacitance which is variable by at least one electrical control signal ($\Delta$U), the capacitance is varied (106) by the at least one electrical control signal ($\Delta$U), and a change ($\Delta$S) of an output signal of the measuring circuit caused by this variation (106) is determined (107).

**8.** The adjustment method according to claim 7, wherein the device (1) contains two components (31, 32), each having a capacitance which is variable by at least one electrical control signal ($\pm\Delta$U), and the capacitances are varied essentially anti-symmetrically to one another (106).

**9.** A use of the adjustment method according to one of the preceding claims for adjusting a yarn clearer.

**10.** A device (1) for examining an elongate textile test material (9), containing a measuring circuit, which is detunable by at least one electrical control signal ($\Delta$U), having a sensor (21) for at least one property of the test material (9),
**characterized by**
an evaluation unit (6) which is configured to execute the adjustment method according to one of the preceding claims.

**11.** The device (1) according to claim 10, wherein the sensor (21) is a capacitive, an optical, and/or a triboelectric sensor.

**12.** The device (1) according to claim 11, wherein the sensor is a measuring capacitor (21) for accommodating the test material (9), and the measuring circuit contains at least one component (31, 32) having a capacitance which is variable by the at least one electrical control signal ($\Delta$U).

**13.** The device (1) according to claim 12, wherein the measuring circuit is designed as a capacitance measuring bridge having at least two bridge branches (11, 12) of which a first bridge branch (11) is a measuring branch, which contains the measuring capacitor (21), and a second bridge branch (12) contains a component (31, 32) having a capacitance which is variable by at least one electrical control signal ($\Delta$U).

**14.** The device (1) according to claim 12 or 13, also containing
means (4) for applying an electrical alternating signal to the measuring circuit for the purpose of generating an electrical alternating field in the measuring capacitor (21), and

a passage opening for the test material (9) in the measuring capacitor (21), to which passage opening the electrical alternating field is applicable.

15. The device (1) according to one of claims 10-14, wherein the device (1) is a yarn clearer.


**Revendications**

1. Procédé de réglage d'un dispositif (1) pour l'examen d'un article textile allongé à contrôler (9),
   lequel dispositif (1) comprend un circuit de mesure pouvant être désaccordé par au moins un signal de commande électrique ($\Delta U$), avec au moins un capteur (21) pour au moins une propriété de l'article à contrôler (9),
   dans lequel le circuit de mesure est désaccordé (101, 106) par l'au moins un signal de commande électrique ($\Delta U$) de façon à donner un changement ($\Delta S$) non nul d'un signal de sortie du circuit de mesure par rapport à une valeur d'équilibrage, provoqué par le désaccord (101, 106),
   **caractérisé en ce que**
   le changement ($\Delta S$) du signal de sortie provoqué par le désaccord (101, 106) est déterminé (102, 107),
   le signal de commande ($\Delta U$) provoquant le désaccord et le changement ($\Delta S$) déterminé du signal de sortie sont associés entre eux (103, 108),
   une valeur de réglage (C) est calculée (104, 109) à partir d'un résultat de l'association (103, 108) et enregistré dans une unité de mémoire du dispositif (1) et
   le réglage est ensuite effectué automatiquement quand aucun article à contrôler (9) ne se trouve dans la zone de mesure du capteur (21).

2. Procédé de réglage selon la revendication 1, dans lequel l'association par le calcul (103, 108) comprend le calcul d'un quotient.

3. Procédé de réglage selon l'une des revendications précédentes, dans lequel la valeur de réglage (C) est proportionnelle ou inversement proportionnelle à un rapport entre le changement déterminé du signal de sortie ($\Delta S$) et le changement produit du signal de commande ($\Delta U$).

4. Procédé de réglage selon la revendication 3 dans lequel une première valeur de réglage (C) et ledit rapport ($\Delta S/\Delta U$) sont déterminés à l'occasion d'un premier réglage (101-104) qui est effectué avec un étalon, et à l'occasion de tous les réglages suivants (106-109), ledit rapport ($\Delta S/\Delta U$) est à nouveau déterminé et une nouvelle valeur de réglage (Cnew) est calculée sur cette base.

5. Procédé de réglage selon l'une des revendications précédentes, dans lequel la valeur d'équilibrage est égale à zéro.

6. Procédé de réglage selon l'une des revendications précédentes, dans lequel l'article à contrôler (9) est un fil.

7. Procédé de réglage selon la revendication 6, dans lequel
   le capteur est un condensateur de mesure (21) destiné à recevoir l'article à contrôler (9),
   le circuit de mesure comprend au moins un composant (31, 32) ayant une capacité qui peut être modifiée par au moins un signal de commande électrique ($\Delta U$),
   la capacité est modifiée (106) par l'au moins un signal de commande électrique ($\Delta U$) et un changement ($\Delta S$) d'un signal de sortie du circuit de mesure provoqué par cette modification (106) est déterminé (107).

8. Procédé de réglage selon la revendication 7, dans lequel le dispositif (1) comprend deux composants (31, 32) ayant chacun une capacité qui peut être modifiée par au moins un signal de commande électrique ($\pm\Delta U$) et les capacités sont modifiées (106) sensiblement de façon antisymétrique l'une par rapport à l'autre.

9. Mise en oeuvre du procédé de réglage selon l'une des revendications précédentes pour le réglage d'un nettoyeur de fil.

10. Dispositif (1) pour l'examen d'un article textile allongé à contrôler (9), comprenant un circuit de mesure qui peut être désaccordé par au moins un signal de commande électrique ($\Delta U$) avec un capteur (21) pour au moins une propriété de l'article à contrôler (9),
    **caractérisé en ce**
    **qu'**il comporte une unité d'interprétation (6) aménagée pour exécuter le procédé de réglage selon l'une des revendications précédentes.

11. Dispositif (1) selon la revendication 10, dans lequel le capteur (21) est un capteur capacitif, optique et/ou triboélectrique.

12. Dispositif (1) selon la revendication 11, dans lequel le capteur est un condensateur de mesure (21) destiné à recevoir l'article à contrôler (9) et le circuit de mesure comprend au moins un composant (31, 32) ayant une capacité qui peut être modifiée par l'au moins un signal de commande électrique ($\Delta U$).

13. Dispositif (1) selon la revendication 12, dans lequel le circuit de mesure est conçu comme un pont de mesure à capacité avec au moins deux branches de pont (11, 12), parmi lesquelles

une première branche de pont (11) est une branche de mesure qui contient le condensateur de mesure (21) et

une deuxième branche de pont (12) est un composant (31, 32) ayant une capacité qui peut être modifiée par au moins un signal de commande électrique ($\Delta$U).

14. Dispositif (1) selon la revendication 12 ou 13, comprenant en outre

des moyens (4) pour appliquer un signal électrique alternatif au circuit de mesure afin de produire un champ électrique alternatif dans le condensateur de mesure (21), et

une ouverture de passage pour l'article à contrôler (9) dans le condensateur de mesure (21), laquelle ouverture de passage peut être exposée au champ électrique alternatif.

15. Dispositif (1) selon l'une des revendications 10 à 14, dans lequel le dispositif (1) est un nettoyeur de fil.

Fig. 1

START

VERSTIMMUNG DURCH ΔU — 101

ERMITTLUNG VON ΔS — 102

$A := \Delta S / \Delta U$ — 103

ANFANGSJUSTIERUNG → C — 104

JUSTIEREN? — 105

NEIN

JA

VERSTIMMUNG DURCH ΔU — 106

ERMITTLUNG VON ΔS — 107

$A_{new} := \Delta S / \Delta U$ — 108

$C_{new} := (A_{new}/A) \cdot C$ — 109

UNTERSUCHUNG — 110

BEENDEN? — 111

NEIN

JA

ENDE

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0924513 A1
- WO 9313407 A1
- EP 1037047 A1
- WO 2006105676 A1
- WO 2007115416 A1
- WO 2010043063 A1
- WO 2010043065 A1